# EUROPEAN PATENT APPLICATION

(11) **EP 0 874 058 A1**
(43) Date of publication of application: **28.10.1998**
(21) Application number: 98500098.3
(22) Date of filing: 21.04.1998
(51) Int. Cl.: C12P 1/04, A61K 35/74, C12N 1/36, C12N 1/06

(54) **Procedure for obtaining a thermoresistant biological extract**

(30) Priority: 24.04.1997 ES 9700887
(71) Applicant: Blajev, Todor Iliev, 28001 Madrid (ES)
(72) Inventor: Blajev, Todor Iliev, 28001 Madrid (ES)
(74) Representative: Urteaga Simarro, José Antonio

(57) **Abstract**

The invention consists in a procedure for the obtaining of a biological extract, with a proteical nature, with thermoresistant proprieties in temperatures between -30 °C (preferably during 18 hours) and +65 °C (preferably during 10 minutes) and the which one is obtained from prokaryote cellules. Said extract possesses immunestimulant properties, which consist in the proliferative induction and activation of T lymphocytes in animal species and in the mankind.

## Description

The invention consists in a procedure for the obtaining of a biological extract, with a proteical nature, with thermoresistant proprieties and which one is obtained from prokaryote cellules. Said extract owns immunestimulant proprieties, which consist in the proliferative induction and activation of T lymphocytes in animal species and in the mankind.

In Immunology, there are distinguished several types of acquired immunity. One of them is obtained by forming a great number of active lymphocytes, specifically programmed for the destruction of the foreign agent (bacterium, virus, etc..). This kind of immunity is denominated immunity mediated by T cellules or cellular immunity.

Actually, they are obtained proteins with immunestimulant and immunemodulating action in animal species and in the mankind. These proteins are named citokins and/or interleukins, but their origin are not prokaryotic cellules and they are not obtained from prokaryotic cellules, excepting an intervention in said cellules by means of genetical insertion through the techniques known as genetic engineering. That is to say, said proteins or peptides did not belong to the type described in the present Patent and they did not own immuneproliferative proprieties of the T lymphocytes.

From another part, they exist proteins known as Granulocytes Stimulant Factor (GSF) and Macrophages Stimulant Factor (MSF), but they do not satisfy in the procedure of obtaining nor in proprieties of the thermoresistant extract, object of the present invention and also due to that they act specifically on other cellules types Granulocytes and Macrophages of the Immune System.

Related to the process of obtaining the FSG and FSM, we reiterate the already exposed in the previous point in relation with the intrinsical non-origin of prokaryotic cellules.

From another part, and within the world of the prokaryotes, some lysates and cellular extracts have been utilised as immunological proprieties products. It is well known the utilisation of Freund's complete Adjuvant (ACF) or incomplete (AF) in order to stimulate the immunological response and provoke the formation of a great number of antibodies mixed with AF or ACF. This kind of response of these lysates is denominated humoral response and, in this way, it doesn't represent the response induced by our thermoresistant extract.

And, for the last, it remains to review in the actual status of the technique, that all the immunologists knows the fact that the bacterium lysates, viral, proteical extracts, etc.. can start an immunological response with a proliferative induction of the total leukocytes. This fact also means the appearance of a quick immunological reaction over the proliferation of B lymphocytes and the probable appearance of allergical reactions and anaphylaxis, if the exposure to these lysates or extracts is realised in repeated doses, in a short term of time (days). In relation with these products our thermoresistant extract did not generates this kind of reactions, due to that it does not induces "in vitro" the polyclonal of B cellules and also due to that it is a thermoresistant extract, characteristic not presented by the rest of the extracts. From another part, if the above mentioned lysates or extracts did not induce any allergical reaction, then the administration of repeated doses (days) does not have the propriety of maintaining in the time the proliferative induction of total leukocytes and these return to the normal ciphers, in dependence of the species. In our case and the mammalian animal species and also in the mankind, the action of proliferative induction of active T lymphocytes is maintained as long as the administration of the thermoresistant extract continues.

Another important particularity is that the above mentioned immunestimulants perform their action through parental via only, while the thermoresistant extract, which we refer to, performs its proliferative action and activating of T lymphocytes per parental administration as well as per oral administration in the same form, even if with higher doses when oral via.

Until today, and in the pathologies of immunedepression or the caused by immunesuppression, in conclusion the pathologies where the organisms needs a reinforcement of the immune system, is suffered the problem of disposing of immunestimulants with immuneproliferative action and activation of the T lymphocytes.

It exists, therefore, the problem of obtaining a "product" capable to induce the proliferation and activation of the T lymphocytes in animal species and also in the mankind, without provoking immediate allergic reactions or delayed hypersensibility, in the major part of the organisms.

This problem has been resolved through the following procedure.

The product is obtained from Sporulated Bacillus, Aerobically, non-pathogenous for the mankind and gram positives which belongs to the species Cereus, circulans, lentus, licheniformis, macerans, megaterium, mesentericus, pumilus y subtilis, without rejecting other sporulated bacillus genus, which are fit to give as result, by means of this procedure, a thermoresistant extract with the proprieties of immuneproliferation and activation of T lymphocytes.

An inoculum containing a strains which belongs to the described species is placed in the culture media, in order to produce its multiplication and growth. As culture media could be utilised any culture media fit for the growth of any bacillus species described previously. The detailed description of the several culture medium described in the scientific literature is not relevant for the purpose of obtaining of the extract, even when any culture media of sporulated aerobical bacillus, non-pathogens for the mankind and which belong to the above described species, is valid. Nevertheless, and as a part of the present Memory, next we will describe the usually utilised for the obtaining of this thermoresistant extract method. The culture media is composed by Meat extract, soluble leaven extract, meat pepsical peptone, meat pancreatic peptone, pancreatic casein peptone, monobasical potassium phosphate, sodium chloride and water, in adequate proportions for the better growth of the bacillus strains which belong to the above-mentioned genus. The pH of the obtained culture media does not need adjustments.

The culture media is sterilised by means of autocode or in the own bioreactor, depending of the case, but minimum at 121ºC, 20 minutes. It is added to the culture media by following the usual microbiological techniques, an inoculum, of strain of bacillus which belongs to one of the above mentioned species. The strains of the different species could be cultivated separately or all together in the same culture medium. In this last case, it exists only the possibility of a litic interference of one of the strains with the rest of them, what is to say that they must be cultivated separately. That is to say, it is possible to cultivate in the same culture media several strains (co-culture) of the above mentioned species if they don't have litic interference, but it is not possible the co-cultivation and the obtaining an adequate performance (bacillus mass) if said litic interference exists. The reason of the co-cultivation is only in order to facilitate the industrial production.

This mixture of inoculum + culture medium is cultivated following the traditional microbiological methods, with the following preferential conditions of cultivation:
Temperature: between 37 to 42 ºC +/- 0.5 ºC (depending of the strain)
Dissolved oxygen: initial 98% +/- 2%, during the culture > 0%
PH: 7.0 +/- 0.2 after reaching pH = 7
Air flow: 0.5 - 1 vvm (air volume per culture media volume)

These conditions are not the unique, any condition which produces the growth of the inoculum and foresees, therefore, as a final result an adequate performance of micro-organisms, expressed as a bacillus mass, is valid. The culture is controlled by Optimal Optical Density, normally 620 nanometers.

The culture is prolonged in these conditions until reaching the stationary phase. This phase could not be longer than 3 hours in order to avoid the sporulation, which one is not limiting but it could interfere in the performance for the obtaining of the extract, when the stationary phase is reached the culture is interrupted and the next step of the process is started.

The obtained result is denominated "growth culture".

For the obtaining of the thermoresistant extract it is proceeded to cellular concentration which one could be obtained through several methods as centrifuging, cutting of filters of selective pore Size, etc.. The result, independently of the utilised procedure, is the obtaining of the "wet cellular mass" which proceeds from the "growth culture".

Next we will describe the normally utilised method for the obtaining of the extract:

The "growth culture" is submitted to cross flow in a 0.22 micrometers pore diameter filter, the range of concentration to obtain is of 10-20 times. That is to say, if we start from an initial volume of 800 litres of culture, 80 to 40 litres of concentrate culture are to obtain. The obtained "non-concentrated culture media" after this process is rejected.

When using this method it is necessary the obtaining an aliquot of the culture before its concentration and to centrifuge to 1000 g/ 15 minutes, reject the liquid (not the pellet) and weight the wet bacillus mass, this data is important in order to, at the end of the process, adjust the final concentration of the extract. It is obvious that the method for knowing the bacillus mass is not very important; it is important to know the weight of the bacillus mass.

Once concentrated the "growth culture", it is proceeded to a diafiltration with the changing of the culture media by a physiological saline solution (ClNa 0.9%). This step is not transcendental for the obtaining of the active extract, but it helps its purifying.

The normally utilised diafiltration means an exchange of 200 litres of physiological saline solution, utilising a membrane filter of 0.22 micrometers.
The obtaining of thermoresistant extract follows the cryothermal, which is described next. The "concentrated culture" (cellular concentration) is placed in cryothermal plastic bags, which are fit to support elevated temperatures without danger for its structure. Actually, for this extract, FLEXboy of 10 litres are utilised. It is indifferent the type of the cryothermal container, but the process must run without troubles.

In this case, the bags, half filled with the cellular concentration, are cooled to refrigerating temperature between 2 and 8 grades centigrade until reaching, in the centre of the cellular mass, said refrigerated temperature and after that it is necessary to submit said masses to a freezing process at a temperature of -5 to -80 grades centigrade, preferably -30 to -40 grades centigrade, during a minimal time of 5 hours to 1 year, preferably 18 hours, and after that - to warm said cellular masses until reaching the temperature of +50 to +85 grades centigrade, preferably +65 grades centigrade, uniformly extended in each mass and maintain said warming during at least 30 seconds to 30 minutes, preferably 10 minutes and repeating the process of freezing and the one of warming 1 to 6 times, preferably 4 times.

This process of freezing - warming could be realised only once, but a contamination by partially thermoresistant structures could be obtained, the one which will not affect the activity of the thermoresistant extract.

As a result of this process it is also produced a cellular cryofracture and the bacillus are lysated, without necessity of enzymatic lysis. A process of enzymatic lysis could be utilised, even when the utilised enzyme doesn't have protein litic capacity on the extract which we obtain at the end of the process, since the active fraction (thermoresistant extract) haves proteical nature, as it is demonstrated by the fact that the activity of the extract is destroyed by the action of certain protein litic enzymes and by the percentage of its concentration of proteins by colour-metric traditional methods and SDS-page. The utilising of enzymatic lysis doesn't excludes the absolute necessity of the above mentioned cold-warm cycle, since the most important part of the whole process is to inactivate, denaturalise, to break those biological structures (proteins, nucleic acids, etc..) which are not thermoresistant to the already described process and which are not constituting the proliferation properties and activation of T lymphocytes. The utilising enzymatic lysis implicates the necessity of eliminating the enzyme once ended its action or of completely inactivate the enzyme.

The result after this process is denominated "Concentrate cellular lysate".

The separation of cellular remains and, therefore, the obtaining of the thermoresistant extract is realised submitting the "concentrated cellular lysate" to homogenisation ultramixer type, obtaining a whitish-yellowish solution, with a milk aspect. The objective of the homogenisation is to liberate the remains of the membrane and cellular oganelles, these proteins thermoresistant to the described process and after that the obtained solution is submitted to cross-flow filtration with diafiltration (exchanging with saline physiological solution) in membrane of cut of 0.22 micrometers. The range of dilution to reach is of 1:10, that is to say if initially there are 100 litres we must obtain 1000 litres of filtering. So, the cellular remains are retained in the filter system and a "filtering" with the wanted components is obtained. The obtained cellular remains retained in the filter are rejected.

The obtained extract is denominated "filtrating". This "filtrating" could be also obtained by centrifuging at more than 5000 g, without utilising the cross-flow filtration. In this case the obtained liquid is diafiltrated in order to realise the exchange with the saline physiological solution. The use of this saline physiological solution is not indispensable but it helps the purifying, because of the removal of non-desirable components.

In this process, for the concentration of the product diluted in the diafiltration of cross-flow (the filtrating), next a system of filter with cut of 5000 units of atomic mass (uma) is utilised, by means of cross-flow filtration of the "filtrating". The obtained range of concentration is of 100:5, that is to say, if we start from 1000 litres we obtain 50 litres. All the molecules, which molecular size is lower or equal to 5000 uma are rejected and are concentrated in a factor 100:5 all these active and thermoresistant to the described process molecules and which molecular weight is higher than 5000 uma.

The so obtained extract is named "the concentrate".

"The concentrate" of the product is filtrated 2 times sterility by a membrane filter of 0.22 micrometers pore diameter.

In conclusion, the objective of this process is to provoke the thermal lysis or enzymatic of the bacillus, nondestructive for the thermoresistant extract and to obtain, through the already mentioned cryothermal process, biological structures of proteical nature, which must be thermoresistant to said process. The obtaining of pharmacological active concentrations with the above mentioned proprieties is realised as it follows:
Of the anterior process, for each used strain, a concentrated liquid is obtained and, therefore, a volume expressed in Litres of concentrated product.
Of the initial culture of each strain or of its co-culture the weight of the wet mass of Bacillus is obtained, by centrifuging in fixed conditions of 1000g/15 and that informs us of how many grams of wet bacillus mass we have in each culture Litre. Multiplying this result by the number of litres of culture, we have the total weight of the wet mass of bacillus per culture. We will denominate this weight "X", we calculate how many litres of physiological saline solution is necessary to add to the concentrate in order to obtain concentrations of 60 grams/ litre, which refer to the wet bacillus mass. We will denominate this concentration 1 unit/millilitre. Obviously, they could be obtained 2, 3, 4, etc.. units/millilitre until reaching the limit of dissolution.
Given that the "concentrate" is obtained in volume and given that said volume of "concentrate" corresponds with its wet bacillus mass, that is to say said volume of concentrate corresponds with its "X", it will have "X"/ Volume of concentrate = number of Kilograms/litre and we will know which is the volume of physiological saline solution to add to the concentrate in order to obtain the wished concentration, in units.
In this way, a biological extract is obtained, which one possesses, to the concentrations described in the experimentation (which we will show next), immunestimulant proprieties of proliferation and activation of T lymphocytes.

Our thermoresistant extract (ET) produces a T-dependant antibodies response, in vivo. To demonstrate it, a technique of "Plaque forming cells essay" is utilised, the experiment, in our case, is realised as it follows: Rats are immunised by means of administrating our thermoresistant extract (ET) in concentration of 120 grams/litre (2 units/millilitre) with a dose of 0.020 to 0.25 millilitres per rat. The rats are also immunised with red sanguine sheep cellules (SRBC) as antigen, then they are sacrificed by means of cervical dislocation and the spleen is surgically extirpated. After 4 days, Immunoglobulins conjugated to SRBC were mixed with lymphocytes, some of them are cellules producing specific antibodies and are incubated in a semisolid culture media in order to permit the secretion of antibodies which joins the surface erythrocyte, next, again, an conjugated to the SRBC Immuneglobuline is added, as a specificity complement. Well-delimited zones of Plaques were measured in order to determine the dependent lymphocyte activation, determining the number of Plaque forming cellules (PCF) for each 1.000.000 of cellules. As a result, the thermoresistant extract (ET) increments the number of T-dependant antibodies, obtaining a dependence dose-response in the SRBC immunised rats. The magnitude of the increasing changes from 200 PCF/10⁶, cellules for the dose of 0.00 millilitres of extract (no-treatment) to 435 PCF/10⁶, cellules for the dose of 0.25 millilitres of thermoresistant extract of 2 units/millilitre of concentration, variations of 215, 320, 345, 365 PCF/10 ⁶, cellules, as it respects, for the doses of 0.020, 0.05, 0.1, 0.2 millilitres of ET. These ciphers, evidently, are submit to the biological variation of any experimentation, but they indicate, without any doubt, that ET increments the number of T-dependant antibodies, producing a dose-dependent response in SRBC immunised rats. In concordance with this result, ET is effective "in vivo" over the immune system in relation with several types of lymphocytes.

Another experience realised "in vitro" shows that ET doesn't presents effects on the proliferation and activation of B cellules. For it, a series of concentrations are added to a splenocytes culture, of ET 2 units/millilitre, that changes from the 1% to the 0.01% of final concentration in the cellular culture. The level of B-dependant antibodies, produced by the cellules, was measured after an in vitro treatment (5 micrograms/millilitre) and without lipopolysaccharide utilising the plaque Forming cells essay FAUCIAS et al; Proc Natl Acad. Sci. USA, oct 1976, 73:101 3676-3679 and FAUCIAS et al, J. Immunol. April 1977, 118:4, 1150-1153. As a result it is obtained that ET doesn't have effect on the proliferation and activation of B cellules.

From another part, ET to the concentration of 2 units/millilitre strongly increments the proliferation of T lymphocytes, as demonstrated using the technique of "Mixed Culture of Lymphocytes in two directions Mangi RJ, Yale J Biol. Med. Jul., 1975 48:3, 217-218". In this technique human lymphocytes are utilised, preferably halogen. The quantification of the response is realised by the determination of tritium- thymidine, which is directly proportional to the DNA synthesis, which last is directly proportional to the level of cellular proliferation. Serial dilutions of ET 2 units/millilitre, varying in its final concentration in the culture from the 1% to the 0.01% are added to the mixture of lymphocytes in culture. After 4 or 5 days, tritium- thymidine is added to the mixture in culture, the culture is advanced during 18-24 hours, the culture media is retired (thymidine not incorporated) and the cellules are collected on a filter paper stripes, the thymidine incorporated into the DNA is counted. Respecting the control (absence of ET) are obtained 7500 DPM/ well, to the dose of 0.01% are obtained 11000 DPM/well, to the dose of 0.1% are obtained 20000 DPM/well and to the dose of 1% are obtained 45000 DPM/well, that is to say a 6 times increment in respecting the control of absence of treatment. As it was already exposed and considering the variability of the biological experimentation, it is concluded that ET strongly increments the proliferation of T lymphocytes, basing it on the mixed culture of lymphocytes in two directions.

In experiments of Blastogenesis, as a final concentration in splenocytes culture, serial dilutions from 1% to 0.01% were utilised (final concentration in culture) of ET 2 units/millilitre. Several series of the cultures were treated, without mitogen, other series with lipopolysaccharide, other one with pokeweed mitogen, other series with phytohemaglutinin, other one with concavalin A, in all the cases with 1 microgram of mitogen/ 250 microlitres of culture, the proliferation of the B and T cellules mediating the trial of incorporation of tritium-thymidine (plaque forming cells assay) was measured. As a result ET intensively increments the response of splenocytes without treating with any mitogen in a range of 10:1, measured as DPM/well, respecting the control (absence of treatment with ET). ET doesn't shows synergism with lipoploysaccharide, which is a mitogen of B cellules. There is some increment, factor 1.5:1 measured as DPM/well, a synergical type with pokeweed mitogen, which one is a T and B cellules common mitogen, to the concentration of the 1% of ET. A synergical effect is presented, in a factor of 2:1, measured as DPM/well and to the concentration of 1% of ET, with concavalinA which one is a T cellules mitogen. ET presents a strong synergism with phytohemaglutinin, in a factor of 5.7:1 measured as DPM/well respecting the control and the concentration of 1% of ET.
The results shows that ET actuates as mitogen by itself, has synergical effects with mitogens of the T cellules, but not with mitogens of the B cellules, that is to say ET owns, in these experiments, a stimulating effect on the T cellules, but not on the B cellules.

In summary, as a conclusion after all the defined experiments and considering the experimental biological variability, ET owns effects over the proliferation and activation of T lymphocytes, but it doesn't provokes effects on the B lymphocytes.

The administration of ET 2 units/millilitre intramuscular and oral via to albino wistar rats, during only 14 consecutive days, administrating 0.1 millilitres/day of ET 2 units/millilitre intramuscular via and 1 millilitre/day of ET 2 units/millilitre oral via, with a intramuscular control group (0.1 millilitre/day of saline physiological solution) and with a oral control group (1 millilitre/day of water) shows an increment in the treated groups respecting the controls of the total leukocytes. The increasing is of 4400/4600 leukocytes / millilitre average in the control groups to 6400/6700 leukocytes/millilitre in the experimental groups. This difference per variation analysis was statistically important. Each group was composed by 10 wistar rats. ET was not toxic in the effectuated analysis, necropsy, organ weight, hemograms, sanguine biochemical and urinalysis. The total lymphocytes also presented an average increasing of 84.5% in the control groups to 89% average in the treated groups, without difference of ET oral or intramuscular treated groups. That is to say, ET causes, in this experiment, an increasing of the total leukocytes and total lymphocytes (expressed as percentage of leukocytes).

In experimentation with humans and VIH positive patients (detected as positives by the technique of immunewesternblot to the virus of the human immunedeficiency) ET increases in a statistically important form its ciphers of T lymphocytes (CD4 and CD8). The T lymphocyte increasing begins, as average, after 2-4 weeks consequent treatment with ET 2 units/millilitre, administrating 2 millilitres each 48 hours intramuscular or subcutaneous via, or 20 millilitres /day oral via. The increasing is very important after 3 months of treatment. The following data, obtained after said experimentation, show the mentioned increasing:
Lymphocytes CD4 (cellules/microlitre) before treatment, after treatment (days)

| | | |
|---|---|---|
| Individual 1 | less than 10 cellules | 110 cellules (84) |
| Individual 2 | 250 cellules | 325 cellules (56) |
| Individual 3 | 400 cellules | 722 cellules (56) |
| Individual 4 | 500 cellules | 600 cellules (56) |
| Individual 5 | 400 cellules | 734 cellules (84) |

Lymphocytes CD8 (cellules/microlitre) before treatment, after treatment (days)

| | | |
|---|---|---|
| Individual 1 | 400 cellules | 875 cellules (84) |
| Individual 2 | 800 cellules | 1 1 0 0 cellules (56) |
| Individual 3 | 1100 cellules | 2250 cellules (56) |
| Individual 4 | 1400 cellules | 1750 cellules (56) |
| Individual 5 | 1300 cellules | 2362 cellules (84) |

On the contrary, in this clinical experimentation, the group without ET treatment, but with antiretroviral treatment (inhibitors of reverse transcriptase) and placebo (saline physiological solution), all the individuals decreased its T lymphocytes ciphers CD4 and CD8) as showed next:
Lymphocytes CD4 (cellules/microlitre) before treatment, after treatment (days)

| | | |
|---|---|---|
| Individual 1 | less than 10 cellules | less than 10 cellules (84) |
| Individual 2 | 300 cellules | 100 cellules (56) |
| Individual 3 | 300 cellules | 100 cellules (84) |
| Individual 4 | 350 cellules | 225 cellules (84) |
| Individual 5 | 50 cellules | less than 10 cellules (84) |

Lymphocytes CD8 (cellules/microlitre) before treatment, after treatment (days)

| | | |
|---|---|---|
| Individual 1 | 70 cellules | 60 cellules (84) |
| Individual 2 | 700 cellules | 625 cellules (56) |
| Individual 3 | 1000 cellules | 980 cellules (56) |
| Individual 4 | 1100 cellules | 875 cellules (56) |
| Individual 5 | 50 cellules | 40 cellules (84) |

Further to this response, T lymphocytary to the treatment, the ET treated individuals and the antiretrovirals, they experimented a clinical melioration, measured in the MOS-HIV scale, contrasting to the individuals which weren't treated with ET, but with antiretrovirals; these last presented a deterioration in the clinical situation, according to the same scale.
The individuals which followed the treatment, longer than 84 days and during 6 months, experimented a continuos increasing of its CD4 and CD8 ciphers until reaching the normal or very close to the normality ciphers, at the same time as their viral cipher in peripheral blood, expressed in Equivalents/millilitres and measured by Branched-ADN suffered in this period drastical reductions of 50000 to 2500 is said units.

## Claims

1. Procedure for the obtaining of a thermoresistant biological extract and characterised by being obtained from microbiological cultures of aerobical sporulated bacillus, gram positives, non-pathogens for the mankind and from any strain which belongs to the species Cereus, Circulans, Lentus, Licheniformis, Macerans, Megaterium, Mesentericus, Pumilus, Stearothermophilus and Subtilis, so that the cellular masses, obtained from the microbiological cultures growth from a inoculum of the strain or strains, are submitted to a cryothermal process, which consists in cooling them to a refrigerating temperature in a range between 2 to 8 grades centigrade until reaching, in the centre of said cellular mass, the above mentioned refrigerating temperature and, as next step, to submit said masses to a freezing process in temperatures between -5 to -80 grades centigrade, preferably -30 to -40 grades centigrade, during a minimal time of 5 hours to 1 year, preferably 18 hours, and, as next step, to warm said cellular masses until reaching the temperature of +50 to +85 grades centigrade, preferably +65 grades centigrade, uniformly distributed in each mass and to maintain said warming during a minimal time of 30 seconds to 30 minutes, preferably 10 minutes, and repeating the process of freezing and warming 1 to 6 times, preferably 4 times, and, as next step, to homogenise said masses by means of an homogenising machine and, as next step, or realising cross flow filtration on 0.22 micrometers or realising centrifuging in order to separate the cellular rests (membranes and cell organelles), preferably to 5000g and, as next step, in one or the another case, to realise an exchange diafiltration with saline physiological solution of the obtained product and, as next step, to realise filtration with a cut filter of 5000 units atomic mass and, as next step, to filter, 2 consecutive times, in sterility trough a filter of 0.22 micrometers, obtaining an thermoresistant biological extract, susceptible of inducing the proliferation of T lymphocytes according to the mixed lymphocytary reaction in two directions, and the no-inducing the proliferation of B lymphocytes according to the trial technique of plaques forming cellules.
